(19) Europäisches Patentamt
European Patent Office
Office européen des brevets

(11) Veröffentlichungsnummer: **0 010 270**
**B1**

(12) # EUROPÄISCHE PATENTSCHRIFT

(45) Veröffentlichungstag der Patentschrift: **04.11.81**

(21) Anmeldenummer: **79103936.5**

(22) Anmeldetag: **12.10.79**

(51) Int. Cl.³: **C 07 D 233/60,** C 07 D 233/61, C 07 D 249/08, A 01 N 43/50, A 01 N 43/64

(54) **Neue alpha-Azolyl-alpha-phenylessigsäurederivate, ihre Herstellung, sie enthaltende Fungizide, Verfahren zur Bekämpfung von Fungi und zur Herstellung von Fungiziden.**

(30) Priorität: **18.10.78 DE 2845293**

(43) Veröffentlichungstag der Anmeldung: **30.04.80 Patentblatt 80/9**

(45) Bekanntmachung des Hinweises auf die Patenterteilung: **04.11.81 Patentblatt 81/44**

(84) Benannte Vertragsstaaten: **AT BE CH DE FR GB IT LU NL SE**

(56) Entgegenhaltungen:
**DE-A-1 925 994**
**DE-A1-2 645 617**

(73) Patentinhaber: **BASF Aktiengesellschaft, Carl-Bosch-Strasse 38, D-6700 Ludwigshafen (DE)**

(72) Erfinder: **Sauter, Hubert, Dr. Dipl.-Chem., Goethestrasse 23, D-6700 Ludwigshafen (DE)**
Erfinder: **Zeeh, Bernd, Dr. Dipl.-Chem., Thorwaldsenstrasse 5, D-6700 Ludwigshafen (DE)**
Erfinder: **Rentzea, Costin, Dr. Dipl.-Ing., Neuenheimer Landstrasse 72, D-6900 Heidelberg (DE)**
Erfinder: **Pommer, Ernst-Heinrich, Dr., Berliner Platz 7, D-6703 Limburgerhof (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 010 270 B1

BUNDESDRUCKEREI BERLIN

# Neue alpha-Azolyl-alpha-phenylessigsäurederivate, ihre Herstellung, sie enthaltende Fungizide, Verfahren zur Bekämpfung von Fungi und zur Herstellung von Fungiziden

Die vorliegende Erfindung betrifft neue, wertvolle α-Azolyl-α-phenylessigsäurederivate, ihre Salze und Metallkomplexe, Verfahren zu ihrer Herstellung sowie ihre Verwendung als Fungizide.

Es ist bekannt, daß Triazol-Derivate, zum Beispiel α-Phenyl-α-1,2,4-triazol-1-yl-essigsäure-t-butylester (DE-OS 2 638 470), gute fungizide Wirksamkeit zeigen, Die Wirkung ist bei niedrigen Aufwandmengen und Anwendungskonzentrationen jedoch nicht immer befriedigend. Darüber hinaus ist die fungitoxische Wirkung oft mit einer hohen Phytotoxizität verbunden, so daß in den für die Bekämpfung von Pilzen im Pflanzenschutz notwendigen Konzentrationen auch die Kulturpflanzen geschädigt werden. Aus diesen Gründen sind sie für den Gebrauch als Pflanzenschutzmittel zur Bekämpfung von Pilzen nicht immer und nicht bei allen Pflanzenarten geeignet.

Es wurden nun Verbindungen gefunden, die gut wirksam gegen Schadpilze, insbesondere aus der Klasse der Ascomyceten und Basidiomyceten sowie Phycomyceten sind.

Gegenstand der Erfindung sind neue α-Azolyl-α-phenyl-essigsäurederivate der Formel I

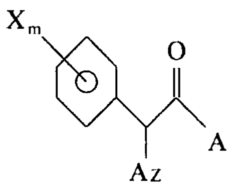

(I)

in der

X Wasserstoff, Fluor, Chlor oder Brom,

m 1, 2 oder 3,

A die Gruppe OR,

worin R für eine Alkyl-, Cycloalkyl-, Alkenyl- oder Alkinylgruppe mit jeweils bis zu 12 Kohlenstoffatomen oder einen Aryl- oder Aralkylrest mit bis zu 12 Kohlenstoffatomen, welcher durch 1 bis 3 Alkyl- oder Alkoxygruppen mit 1–4 Kohlenstoffatomen, Halogenatome, Trifluormethyl-, Cyano- und/oder Nitrigruppen substituiert sein kann, steht, oder

$NR^1R^2$,

worin $R^1$ und $R^2$ gleich oder verschieden sind und für Wasserstoffatome, Alkyl-, Cycloalkyl-, Alkenyl- oder Alkinylgruppen mit jeweils bis zu 12 Kohlenstoffatomen oder Aryl- oder Aralkylgruppen mit jeweils bis zu 12 Kohlenstoffatomen. die durch 1–3 Alkyl- oder Alkoxygruppen mit 1–4 Kohlenstoffatomen, Halogenatome, Trifluormethyl-, Cyano- und/oder Nitrogruppen substituiert sein können, stehen oder $R^1$ und $R^2$ zusammen mit N einen gesättigten Ring mit 4–8 Ringgliedern bilden, der zusätzlich ein Sauerstoff- oder Schwefelatom enthalten und durch 1–4 Halogenatome oder Alkylgruppen mit 1–4 Kohlenstoffatomen substituiert sein kann, und

Az einen Imidazol-1-yl oder 1,2,4-Triazol-1-yl-rest bedeuten,

wobei jedoch A ein Rest $NR^1R^2$ sein muß, worin $R^1$ und $R^2$ keine Wasserstoffatome sind, wenn X Wasserstoff ist,

sowie deren Säureadditionssalze und Metallkomplexe.

R bedeutet beispielsweise Methyl, n-Propyl, Isopropyl, t-Butyl, t-Amyl, n-Octyl, Cyclohexyl, Allyl, Propargyl, Benzyl, 4-Chlorbenzyl.

$R^1$ und $R^2$ bedeuten beispielsweise Wasserstoff, Methyl, Ethyl, n-Propyl, Isopropyl, n-Butyl, Isobutyl, t-Butyl, Isopentyl, t-Amyl, n-Hexyl, 2-Ethylhexyl, Cyclohexyl, Allyl, Propargyl, 1-Butin-3-yl, Phenyl, 4-Chlorphenyl, 2,4-Dichlorphenyl, 4-Fluorphenyl, 2-Fluorphenyl, 4-Bromphenyl, 3-Trifluormethylphenyl, Benzyl, 4-Chlorbenzyl, 2,4-Dichlorbenzyl, 4-Fluorbenzyl.

$R^1$ und $R^2$ können zusammen mit N auch einen gesättigten Ring bilden; in diesen Fällen bedeutet A beispielsweise Pyrrolidino, Piperidino, 2,6-Dimethylpiperidino, Morpholino, 2,6-Dimethylmorpholino.

Unter den Wirkstoffen der Formel I sind zwei Gruppen bevorzugt: Die erste Gruppe umfaßt die Verbindungen, in denen $X_m$ 2-Cl, 4-Cl oder 2,4-$Cl_2$ bedeutet und A und Az die angegebene Bedeutung haben. Zur zweiten Gruppe gehören die Verbindungen, in denen A $NR^1R^2$ bedeutet, wobei jedoch $R^1$ und $R^2$ nicht für Wasserstoff stehen, und X, m und Az die angegebene Bedeutung haben.

Säureadditionssalze sind beispielsweise die Hydrochloride, Bromide, Sulfate, Nitrate, Phosphate, Oxalate oder Dodecylbenzolsulfonate. Die Wirksamkeit der Salze geht auf das Kation zurück, so daß die Wahl des Anions beliebig ist.

Metallkomplexe sind Verbindungen der Formel

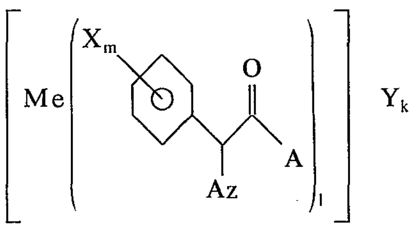

(II)

in der X, m, A und Az die oben angegebene Bedeutung haben und

Me ein Metall, z. B. Kupfer, Zink, Zinn, Mangan, Eisen, Cobalt oder Nickel bedeutet,
Y für das Anion einer anorganischen Säure steht, z. B. Salzsäure, Schwefelsäure, Phosphorsäure oder Bromwasserstoffsäure und
l und k 1, 2, 3 oder 4 bedeuten.

Gegenstand der Erfindung ist weiter ein Verfahren zur Herstellung der Verbindungen der Formel I, welches darin besteht, daß man α-Halogencarbonsäurederivate der Formel III

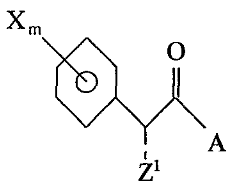

(III)

in der X, m und A die oben angegebene Bedeutung haben und $Z^1$ ein Chlor- oder Bromatom bedeutet, mit den Azolen HAz, in denen Az die oben angegebene Bedeutung hat, gegebenenfalls in Gegenwart einer Base und/oder eines Lösungs- oder Verdünnungsmittels umsetzt.

Zur Herstellung der neuen Verbindungen ist es zweckmäßig, die α-Halogencarbonsäurederivate der Formel III in An- oder Abwesenheit eines Lösungs- oder Verdünnungsmittels mit etwa 0,5 bis 2 Äquivalenten eines Alkalisalzes des jeweiligen Azols, gegebenenfalls unter Zusatz einer Base bei Temperaturen von etwa 0 bis 200°C, vorzugsweise +20°C bis 160°C in homogener oder inhomogener Phase umzusetzen. Als Lösungs- bzw. Verdünnungsmittel können z. B. Methanol, Ethanol, Isopropanol, n-Butanol, Diethylether, Tetrahydrofuran, Dioxan, Dimethylsulfoxid, Chloroform, Methylenchlorid, Toluol und vorzugsweise Aceton, Acetonitril oder Dimethylformamid verwendet werden. Als Basen können z. B. organische Amine wie Triethylamin, Pyridin oder anorganische Verbindungen wie z. B. Kaliumcarbonat oder Natriumhydroxid verwendet werden.

Die als Ausgangsstoffe verwendeten Verbindungen der Formel III lassen sich durch Acylierung eines Alkohols der Formel HOR bzw. eines Amins der Formel $HNR^1R^2$, in denen R, $R^1$ und $R^2$ die oben angegebene Bedeutung haben, mit den α-Halogencarbonsäurehalogeniden der Formel

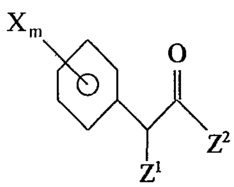

(IV)

in der X, m und $Z^1$ die oben angegebene Bedeutung haben und $Z^2$ ein Chlor- oder Bromatom ist, nach an sich bekannten Methoden (siehe z. B. »Organikum«, Kap. 7.1.5.1 und Kap. 7.1.5.2, VEB Deutscher Verlag der Wissenschaften, 14. Aufl., Berlin 1975) herstellen.

Die α-Halogencarbonsäurehalogenide der Formel IV, sind durch Umsetzung der entsprechenden Mandelsäuren nach den üblichen Halogenierungsverfahren, z. B. durch Umsetzung mit Thionylchlorid, Thionylbromid, Phosphorylchlorid oder Phosphorpentachlorid zugänglich.

Die Verbindungen der Formel IV sind ferner aus Phenylessigsäuren durch Umsetzung zu den entsprechenden Säurehalogeniden, z. B. mit Thionylchlorid, Thionylbromid oder Phosphortribromid, und anschließende Einführung von $Z^1$ durch Reaktion mit Brom, Chlor, Sulfurylchlorid oder N-Bromsuccinimid nach bekannten Methoden zugänglich (siehe E. Schwenk, D. Papa, J. Amer. Chem. Soc. 70, 3626 (1948); P. Truitt, D. Mark, L. M. Long, J. Jeanes, ibid. 70, 4214 (1948)).

Die erfindungsgemäßen Wirkstoffe der Formel I lassen sich mit Säuren in üblicher Weise in ihre Salze, z. B. Hydrochloride, Sulfate, Nitrate, Oxalate, Formiate, Acetate oder Dodecylbenzolsulfonate überführen.

Die Wirkstoffe der Formel I lassen sich außerdem in die Metallkomplexe der Formel II überführen, wenn man sie mit bekannten Metallsalzen der Formel

$$MeY_k \cdot aH_2O \qquad (V)$$

in welcher
Me, Y und k die oben angegebene Bedeutung haben und a 0, 1, 2, 3 oder 4 bedeutet, in Gegenwart eines Lösungsmittels umsetzt. Hier steht Me vorzugsweise für Metalle der I., II. und IV. bis VII. Nebengruppe des Periodensystems der Elemente sowie für Metalle der II. und IV. Hauptgruppe, insbesondere für Kupfer, Zink, Zinn, Mangan, Eisen, Cobalt oder Nickel.

Für die Herstellung der Metall-Komplexe der Formel II kommen alle mit Wasser mischbaren Lösungsmittel in Frage. Hierzu gehören vorzugsweise Methanol, Ethanol, Isopropanol, Aceton, Tetrahydrofuran und Dioxan. Dabei arbeitet man im allgemeinen bei Temperaturen zwischen 0° und 100°C, vorzugsweise zwischen 10 und 35°C.

In den erfindungsgemäßen Wirkstoffen der Formel I ist das azolylsubstituierte Kohlenstoffatom chiral; die Wirkstoffe fallen demgemäß als Enantiomerengemische an, die in die einzelnen Enantiomere getrennt werden können. Falls auch der Teil A der Formel I ein oder mehrere chirale Zentren enthält, treten im Gemisch zusätzlich Diastereomere auf, die sich in üblicher Weise, z. B. durch Chromatographie oder Kristallisation in die einzelnen diastereomeren Komponenten trennen lassen. Für die Anwendung der Wirkstoffe als Fungizide ist jedoch eine Trennung der Enantiomeren oder Diastereomeren normalerweise nicht erforderlich.

Die folgenden Beispiele schildern die Herstellung der neuen Substanzen.

## Herstellung der Ausgangsstoffe

a) Eine Mischung von 88,4 g 2,4-Dichlormandelsäure, 120 ml Toluol, 1 ml Dimethylformamid und 143 g Thionylchlorid wird unter Rühren bis zur Beendigung der Gasentwicklung auf 50°C erhitzt. Nach Abdampfen des Toluols und des überschüssigen Thionylchlorids im Vakuum destilliert man den Rückstand zweimal über eine kurze Vigreux-Kolonne. Die bei 85—95°C/0,13 mbar übergehenden Fraktionen liefern 45 g α-Chlor-α-2,4-dichlorphenyl-acetylchlorid.

$^1H$—NMR (60 MHz, $CDCl_3$):
δ=6,0 (1 H, s), 7,1—7,6 ppm (3 H, s).
IR (Film):
1795, 1584, 1472, 1100, 1043, 982, 864, 776, 736, 693 $cm^{-1}$.
Analog erhält man:
α-Chlor-α-(4-chlorphenyl)-acetylchlorid, Kp. 105—115°C/0,53 mbar.
$^1H$—NMR (60 MHz, $CDCl_3$):
δ=5,6 (1 H, s), 7,4 ppm (4 H, m).
IR (Film):
1787, 1588, 1488, 1202, 1090, 1013, 980, 740, 690 $cm^{-1}$.

b) Zu der auf 5°C gekühlten Lösung von 62 g α-Chlor-α-(2,4-dichlorphenyl)-acetylchlorid in 60 ml Dichlormethan tropft man unter Rühren und Eiskühlung eine Lösung von 17,6 g t-Butanol und 19,2 ml Pyridin in 60 ml Dichlormethan so zu, daß die Temperatur der Reaktionsmischung unter 10°C bleibt. Nach Beendigung des Zutropfens wird über Nacht bei Raumtemperatur nachgerührt, die Reaktionsmischung sodann mit 100 ml Dichlormethan verdünnt und fünfmal mit je 100 ml Wasser extrahiert. Die über Magnesiumsulfat getrocknete organische Phase wird nach Abdampfen des Lösungsmittels destilliert. Man erhält 58 g α-Chlor-α-(2,4-dichlorphenyl)-essigsäure-t-butylester als farbloses Öl vom Kp. 98—103°C/0,13 mbar.

$^1H$—NMR (60 MHz, $CDCl_3$):
δ=1,4 (9 H, s), 5,7 (1 H, s), 7,2—7,8 (3 H, m).

Entsprechend werden die folgenden Ester hergestellt:

α-Chlor-α-(4-chlorphenyl)-essigsäure-t-butyl-ester, Kp. 100°C/0,27 mbar,
α-Chlor-α-(2,4-dichlorphenyl)-essigsäure-t-amyl-ester.

c) Unter Rühren tropft man zur Lösung von 22,4 g α-Chlor-α-(2,4-dichlorphenyl)-acetylchlorid (siehe a)) in 100 ml Dioxan 15,2 g N-2-Butyl-N-methylamin. Nach Abklingen der exothermen Reaktion rührt man über Nacht nach, gießt die Mischung sodann in 700 ml Eiswasser, säuert mit verdünnter Salzsäure an und extrahiert das ausgefallene Öl achtmal mit je 100 ml Dichlormethan. Aus den vereinigten Extrakten bleiben nach dem Trocknen über Magnesiumsulfat und Einengen 17,2 g α-Chlor-α-(2,4-dichlorphenyl)-essigsäure-N-2-butyl-N-methyl-amid als farbloses Öl.

$^1H$—NMR (60 MHz, $CDCl_3$):
δ=0,5—1,8 (8 H, m), 2,7—3,0 (3 H, 4 Singuletts), 3,2—4,9 (1 H, m), 7,2—7,9 (3 H, m): 4 diastereomere und rotamere Komponenten.

d) Zur Lösung von 24,5 g α-Chlor-α-(4-chlorphenyl)-acetylchlorid (siehe a) in 50 ml Dioxan tropft man unter Rühren 22,3 g Diisopropylamin. Nach Abklingen der exothermen Reaktion rührt man über Nacht nach, gießt die Mischung sodann in 700 ml Eiswasser, säuert mit verdünnter Salzsäure an und extrahiert das abgeschiedene Öl achtmal mit je 100 ml Dichlormethan. Die vereinigten Extrakte werden zweimal mit Natriumcarbonatlösung gewaschen, über Magnesiumsulfat getrocknet und eingeengt. Man erhält 27,2 g α-Chlor-α-(4-chlor-phenyl)-essigsäure-diisopropylamid als bräunliches Öl.

$^1H-NMR$ (270 MHz, $CDCl_3$):

$\delta=0{,}95$ (3 H, d), 1,15 (3 H, d) 1,40 (3 H, d), 1,45 (3 H, d), 3,45 (1 H, m), 4,00 (1 H, m), 5,65 (1 H, s), 7,40 ppm (4 H, AA'BB').

IR (Film):

2965, 1648, 1489, 1448, 1369, 1320, 1088, 1039, 1013, 761 $cm^{-1}$.

Entsprechend c und d werden die in Tabelle 1 aufgeführten Amide der Formel III hergestellt, worin $Z^1$ ein Chloratom darstellt. Die Verbindungen werden im allgemeinen durch ihre Folgeprodukte der Formel I charakterisiert. (Die Nr. der Substanzen beziehen sich in allen Tabellen auf die Beispiele).

Tabelle 1

| $X_m$ | – A | Charakterisiert durch Verbindung Nr. |
|---|---|---|
| 2,4-$Cl_2$ | 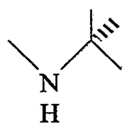 | 3 |
| 2,4-$Cl_2$ | 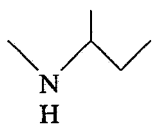 | 4 |
| 2,4-$Cl_2$ | 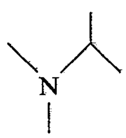 | 5 |
| 2,4-$Cl_2$ | 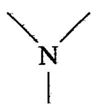 | 6 |
| 2,4-$Cl_2$ | 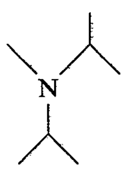 | 7 |
| 2,4-$Cl_2$ | 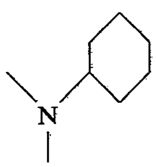 | 8 |
| 2,4-$Cl_2$ | 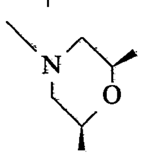 | 9 |
| 2,4-$Cl_2$ | 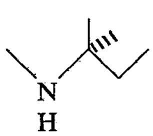 | 10 |

Fortsetzung

| $X_m$ | – A | Charakterisiert durch Verbindung Nr. |
|---|---|---|
| 2,4-$Cl_2$ | 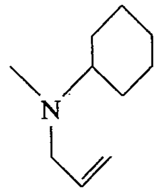 | 17 |
| 2,4-$Cl_2$ | 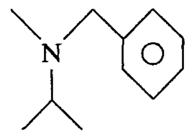 | 18 |
| 2,4-$Cl_2$ | 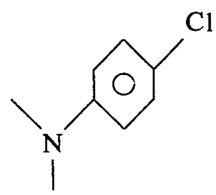 | 20 |
| 2,4-$Cl_2$ | 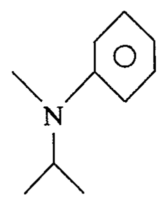 | 21 |

## Herstellung der Endprodukte

### Beispiel 1

α-(2,4-Dichlorphenyl)-α-(1,2,4-triazol-1-yl)-essigsäure-N-2-butyl-N-methyl-amid

Zur Suspension von 1,6 Natriumhydrid in 50 ml Dimethylformamid tropft man unter Rühren 5,4 g 1,2,4-Triazol in 30 ml Dimethylformamid und rührt noch ca. 1 Stunde bis zur Beendigung der Gasentwicklung. Dann werden 17,2 g α-Chlor-α-(2,4-dichlorphenyl)-essigsäure-N-2-butyl-N-methyl-amid zugetropft und nach Abklingen der exothermen Reaktion noch 15 Stunden nachgerührt. Die Mischung wird in 300 ml Dichlormethan aufgenommen und viermal mit je 100 ml Wasser extrahiert. Nach Trocknung der organischen Phase und Abdampfen des Lösungsmittels im Vakuum bleibt ein helles Öl zurück, aus dem sich beim Anreiben mit Diisopropylether 9,8 g farblose Kristalle vom Fp. 116–119° abscheiden.

$^1H$–NMR (220 MHz, $CDCl_3$):
$\delta$=0,4–1,7 (8 H, mehrere Multipletts), 2,7–2,9 (3 H, m), 3,2–3,8 und 4,5–4,8 (1 H, 2 Multipletts), 6,8–6,9 (1 H, m), 7,2–7,6 (3 H, m), 7,9–8,1 ppm (2 H, m): 4 rotamere und diastereomere Komponenten.

### Beispiel 2

α-(4-Chlorphenyl)-α-imidazol-1-yl-essigsäure-diisopropylamid

Zur Suspension von 1,3 g Natriumhydrid in 30 ml Dimethylformamid tropft man unter Rühren eine Lösung von 4,5 g Imidazol in 30 ml Dimethylformamid. Nach Beendigung der Gasentwicklung tropft man eine Lösung von 13,6 g α-Chlor-α-(4-chlorphenyl)-essigsäure-diisopropylamid (siehe d) in 100 ml Dimethylformamid zu und rührt über Nacht bei Raumtemperatur nach. Das nach Abdampfen des Lösungsmittels verbleibende Öl wird in 100 ml Dichlormethan aufgenommen und fünfmal mit je 100 ml 5 proz. Natriumchloridlösung ausgeschüttelt. Die organische Phase wird sodann eingeengt und über Kieselgel (25 x 6 cm) mit Dichlormethan/Aceton 9 : 1 chromatographiert. Nach Abtrennung von 2 l Vorlauf erhält man die Produktfraktionen, aus denen nach dem Einengen und Anreiben mit 10 ml

Diisopropylether 8,9 g farblose Kristalle vom Fp. 112° C isoliert werden.

$^1H$ – NMR (270 MHz, $CDCl_3$):

0,9 (d, 3 H), 1,2 (d, 3 H), 1,5 (2 d, 6 H), 3,5 (m, 1 H), 3,9 (m, 1 H), 6,1 (s, 1 H), 6,9 (s, 1 H), 7,0 (s, 1 H), 7,2 – 7,4 (AA'BB', 4 H), 7,5 ppm (s, 1 H).

Analog lassen sich herstellen:

| Nr. | $X_m$ | Az | –A | Fp (°C) |
|---|---|---|---|---|
| 3 | 2,4-$Cl_2$ | 1,2,4-Triazol-1-yl | 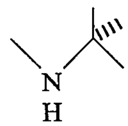 | 167–171 |
| 4 | 2,4-$Cl_2$ | 1,2,4-Triazol-1-yl | 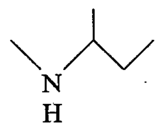 | 129–132 |
| 5 | 2,4-$Cl_2$ | 1,2,4-Triazol-1-yl | 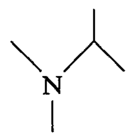 | 104–107 |
| 6 | 2,4-$Cl_2$ | 1,2,4-Triazol-1-yl | 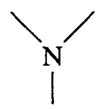 | 115–119 |
| 7 | 2,4-$Cl_2$ | 1,2,4-Triazol-1-yl | 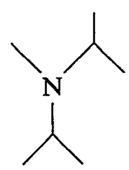 | 117–120 |
| 8 | 2,4-$Cl_2$ | 1,2,4-Triazol-1-yl | 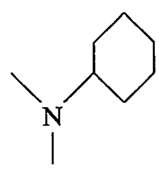 | 157–161 |
| 9 | 2,4-$Cl_2$ | 1,2,4-Triazol-1-yl | 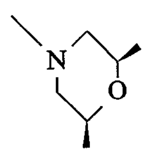 | 131–134 |
| 10 | 2,4-$Cl_2$ | 1,2,4-Triazol-1-yl | 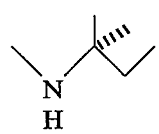 | 156–157 |
| 11 | 2,4-$Cl_2$ | Imidazol-1-yl | 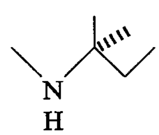 | 123–126 |
| 12 | 4-Cl | 1,2,4-Triazol-1-yl | 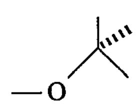 | 92 |

(Fortsetzung)

| Nr. | $X_m$ | Az | – A | Fp (°C) |
|---|---|---|---|---|
| 13 | 2,4-$Cl_2$ | 1,2,4-Triazol-1-yl | 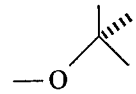 | 83–85 |
| 14 | 2,4-$Cl_2$ | 1,2,4-Triazol-1-yl | 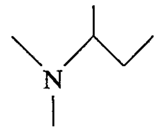 | 116–119 |
| 15 | 2,4-$Cl_2$ | 1,2,4-Triazol-1-yl | 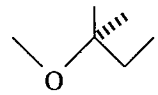 | IR (Film): 2965, 1745, 1465, 1260, 1130, 1002, 865, 812, 672 $cm^{-1}$ |
| 16 | 2,4-$Cl_2$ | 1,2,4-Triazol-1-yl | 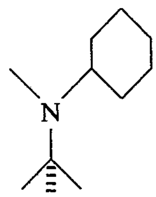 | 121–123 |
| 17 | 2,4-$Cl_2$ | 1,2,4-Triazol-1-yl | 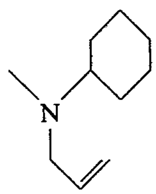 | 111–112 |
| 18 | 2,4-$Cl_2$ | 1,2,4-Triazol-1-yl | 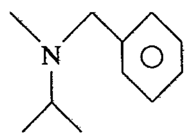 | 93–97 |
| 19 | 2,4-$Cl_2$ | 1,2,4-Triazol-1-yl | 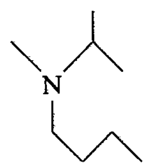 | 81–82 |
| 20 | 2,4-$Cl_2$ | 1,2,4-Triazol-1-yl | 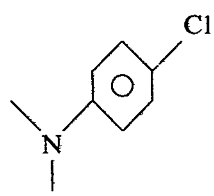 | IR (Film): 1670, 1483, 1380, 1271, 1131, 1096, 1087, 1008, 799 $cm^{-1}$ |
| 21 | 2,4-$Cl_2$ | 1,2,4-Triazol-1-yl | 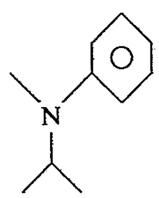 | 130–131 |
| 22 | 2,4-$Cl_2$ | 1,2,4-Triazol-1-yl | 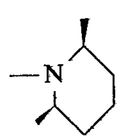 | 148–151 |

(Fortsetzung)

| Nr. | $X_m$ | Az | – A | Fp (°C) |
|---|---|---|---|---|
| 23 | 2,4-$Cl_2$ | 1,2,4-Triazol-1-yl | 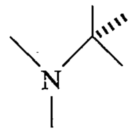 | 99–101 |
| 24 | 4-Cl | 1,2,4-Triazol-1-yl | 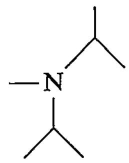 | 123 |
| 25 | 4-Cl | Imidazol-1-yl | 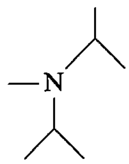 | 112 |
| 26 | 4-Cl | 1,2,4-Triazol-1-yl | 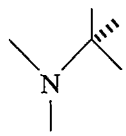 | 115 |
| 27 | 4-Cl | Imidazol-1-yl | 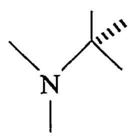 | IR (Film): 2950, 1650, 1484, 1361, 1220, 1068, 1010, 795, 658 $cm^{-1}$ |
| 28 | 4-Cl | 1,2,4-Triazol-1-yl | 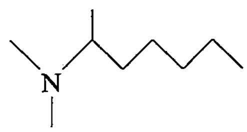 | IR (Film): 2917, 1640, 1431, 1272, 1138, 675 $cm^{-1}$ |
| 29 | 4-Cl | 1,2,4-Triazol-1-yl | 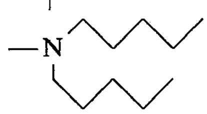 | 2945, 2920, 1650, 1489, 1458, 1271, 1131, 1012, 793, 675 $cm^{-1}$ |
| 30 | H | 1,2,4-Triazol-1-yl | 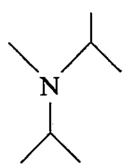 | 106 |
| 31 | H | 1,2,4-Triazol-1-yl | 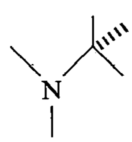 | 115 |
| 32 | H | 1,2,4-Triazol-1-yl | 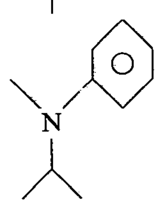 | IR (Film): 2961, 1659, 1490, 1393, 1272, 1112, 724, 702, 675 $cm^{-1}$ |
| 33 | H | 1,2,4-Triazol-1-yl | 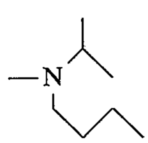 | IR (Film): 2950, 1650, 1494, 1450, 1419, 1272, 1197, 1127, 752, 676 $cm^{-1}$ |

(Fortsetzung)

| Nr. | $X_m$ | Az | – A | Fp (°C) |
|---|---|---|---|---|
| 34 | 4-Cl | 1,2,4-Triazol-1-yl | 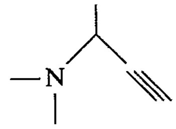 | IR (Film): 3290, 2920, 1655, 1490, 1274, 1088, 1014, 672 $cm^{-1}$ |
| 35 | 4-Cl | 1,2,4-Triazol-1-yl | 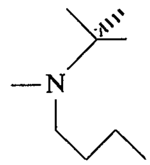 | 102–105 |
| 36 | 4-Cl | 1,2,4-Triazol-1-yl | 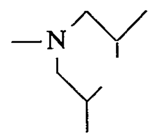 | 60–62 |
| 37 | 4-Cl | Imidazol-1-yl | 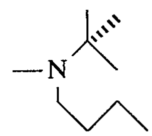 | 89–91 |
| 38 | 2-Cl | 1,2,4-Triazol-1-yl | 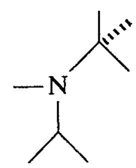 | |
| 39 | 2-Cl | 1,2,4-Triazol-1-yl | 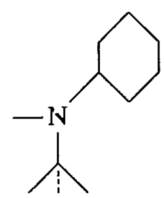 | |
| 40 | 2-Cl | 1,2,4-Triazol-1-yl | 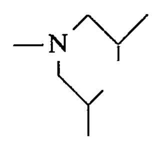 | 130–132 |
| 41 | 2-Cl | 1,2,4-Triazol-1-yl | 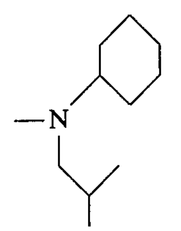 | |
| 42 | 2-Cl | 1,2,4-Triazol-1-yl | 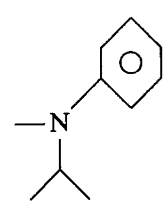 | |

Die neuen Verbindungen und ihre Salze oder Metallkomplexe zeigen eine breite fungizide Wirkung und eine sehr gute Pflanzenverträglichkeit.

Unter den neuen Wirkstoffen der Formel I sind zwei Gruppen besonders bevorzugt. Die erste bevorzugte Gruppe umfaßt diejenigen Verbindungen der Formel I, in denen $X_m$=2,4-Dichlor bedeuten und in denen A und Az die oben genannten Bedeutungen haben. Die zweite bevorzugte Gruppe umfaßt diejenigen Verbindungen der Formel I, in denen $A=NR^1R^2$ bedeutet, worin $R^1$ und $R^2$ nicht für Wasserstoff stehen und in denen X, m und Az die angegebene Bedeutung haben.

Die neuen Wirkstoffe können auch in Form ihrer Salze und Metallkomplexe verwendet werden.

Besonders eignen sich die erfindungsgemäßen Verbindungen zur Bekämpfung von Pilzerkrankungen an verschiedenen Kulturpflanzen, z. B. bei Ustilago sctiaminea (Zuckerrohrbrand), Hemileia vastatrix (Kaffeerost), Uromyces fabae bzw. appandiculatus (Bohnenrost), Rhizoctonia solani, Erysiphe graminis (Getreidemehltau), Uncinula necator, Sphareotheca fuliginea, Erysiphe cichoracearum, Podosphaera leucotricha, Venturia ınaequalis (Apfelschorf), Plasmopara viticola (Rebenperonospora), Pseudoperonospora humuli.

Unter Kulturpflanzen verstehen wir in diesem Zusammenhang insbesondere Weizen, Roggen, Gerste, Hafer, Reis, Mais, Apfelbaum, Gurken, Bohnen, Kaffee, Zuckerrohr, Weinrebe, Erdbeeren sowie Zierpflanzen im Gartenbau.

Die erfindungsgemäßen Wirkstoffe sind systematisch wirksam. Die systemische Wirksamkeit dieser Mittel ist von besonderem Interesse im Zusammenhang mit der Bekämpfung von inneren Pflanzenkrankheiten, z. B. Getreidemehltau.

Die erfindungsgemäßen Mittel können gleichzeitig das Wachstum von zwei oder mehr der genannten Pilze unterdrücken und besitzen eine hohe Pflanzenverträglichkeit. Die zur Bekämpfung der phytopathogenen Pilze erforderlichen Aufwandmengen liegen zwischen 0,05 und 2 kg Wirkstoff/ha Kulturfläche.

Die erfindungsgemäßen Wirkstoffe können in die üblichen Formulierungen übergeführt werden, wie Lösungen, Emulsionen, Suspensionen, Pulver, Pasten und Granulate. Diese werden in bekannter Weise hergestellt, z. B. durch Vermischen des Wirkstoffs mit Lösungsmitteln und/oder Trägerstoffen, ggf. unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Ethanolamin, Dimethylformamid) und Wasser; Trägerstoffe wie natürliche Gesteinsmehle (z. B. Kaoline, Tonerden, Talkum, Kreide) und synthetische Gesteinsmehle (z. B. hochdisperse Kieselsäure, Silikate); Emulgiermittel, wie nichtionogene und anionische Emulgatoren (z. B. Polyoxyethylen-Fettalkohol-Ether, Alkylsulfonate und Arylsulfonate) und Dispergiermittel, wie Lignin, Sulfitablaugen und Methylcellulose.

Die Formulierungen enthalten im allgemeinen zwischen 0,1 und 95 Gew.-% Wirkstoff, vorzugsweise zwischen 0,5 und 90%. Die Formulierungen, bzw. die daraus hergestellten, gebrauchsfertigen Zubereitungen wie Lösungen, Emulsionen, Suspensionen, Pulver, Pasten oder Granulate, werden in bekannter Weise angewendet, beispielsweise durch Versprühen, Vernebeln, Verstäuben, Verstreuen, Beizen oder Gießen.

Beispiele für solche Formulierungen sind:

I. Man vermischt 90 Gewichtsteile der Verbindung 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung 13 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in 10 000 Gew.-Teilen Wasser erhält man eine wäßrige Dispersion, die 0,02 Gew.-% des Wirkstoffs enthält.

III. 20 Gew.-Teile der Verbindung 16 werden in einer Mischung gelöst, die aus 40 Gew.-Teilen Cyclohexanon, 30 Gewichtsteilen Isobutanol, 20 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.

IV. 20 Gew.-Teile der Verbindung 22 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.

V. 20 Gew.-Teile der Verbindung 21 werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfitablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.-% des Wirkstoffs enthält.

VI. 3 Gew.-Teile der Verbindung 16 werden mit 97 Gew.-Teilen feinteiligem Kaolin innig vermischt. Man erhält auf diese Weise ein Stäubemittel, das 3 Gew.-% des Wirkstoffs enthält.

VII. 30 Gew.-Teile der Verbindung 20 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile des Wirkstoffs 17 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit 100 000 Gew.-Teilen Wasser erhält man eine wäßrige Dispersion, die 0,04 Gew.-% Wirkstoff enthält.

IX. 20 Teile des Wirkstoffs 3 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, z. B. Herbiziden, Insektiziden, Wachstumsregulatoren und Fungiziden oder auch mit Düngemitteln vermischt werden. Beim Vermischen mit Fungiziden erhält man dabei in vielen Fällen eine Vergrößerung des Wirkungsspektrums und eine Leistungssteigerung (Synergismus).

Das folgende Beispiel A zeigt die biologische Wirkung der neuen Substanzen. Als Vergleichssubstanzen dienten die aus der DE-OS 2 638 470 bekannten Verbindungen α-Phenyl-α-1,2,4-triazol-1-yl-essigsäure-t-butylester (Y) und α-Phenyl-α-1,2,4-triazol-1-yl-essigsäure-t-butylamid (Z).

## Beispiel A

Blätter von in Töpfen gewachsenen Weizensämlingen der Sorte »Caribo« werden mit wäßrigen Emulsionen aus 80% (Gew.-%) Wirkstoff und 20% Emulgiermittel besprüht und zwei Tage nach dem Antrocknen des Spritzbelages mit Aidien (Sporen) des Weizenmehltaus (Erysiphe graminis var. tritici) bestäubt. Die Versuchspflanzen werden anschließend in einer Vegetationshalle bei Temperaturen zwischen 18 und 24° C aufgestellt. Nach 10 Tagen wird das Ausmaß der Mehltaupilzentwicklung ermittelt.

| Wirkstoff | Befall der Blätter nach Spritzung mit . . . %iger Wirkstoffbrühe 0,025 | 0,012 | 0,006 |
|---|---|---|---|
| 5 | 0 | 0 | 1 |
| 7 | 0 | 0 | 0 |
| 8 | 1 | 1 | 1 |
| 13 | 0 | 0 | 0 |
| 14 | 0 | 0 | 4 |
| 15 | 0 | 0 | 3 |
| 16 | 0 | 0 | 0 |
| 17 | 0 | 0 | 2 |
| 18 | 0 | 0 | 2 |
| 19 | 0 | 0 | 0 |
| 20 | 0 | 0 | 0 |
| 21 | 0 | 0 | 0 |
| 22 | 0 | 0 | 0 |
| 23 | 0 | 0 | 0 |
| 26 | 1 | 2 | 3 |
| 30 | 0 | 0 | 0 |
| 31 | 0 | 1 | 3 |
| 32 | 0 | 2 | 2 |
| 34 | 0 | 0 | 0 |
| 35 | 0 | 0 | 0 |
| 36 | 0 | 0 | 0 |
| 37 | 0 | 0 | 1 |
| 42 | 0 | 0 | 0 |
| Y | 4 | 4 | 5 |
| Z | 2 | 3 | 4 |
| Kontrolle (unbehandelt) | 5 | | |

0 = kein Pilzwachstum.
Abgestuft bis 5 = Blätter total befallen.

## Beispiel B

Blätter von Topfreben der Sorte Müller-Thurgau werden mit wäßrigen Suspensionen, die 80% (Gewichtsprozent) des zu prüfenden Wirkstoffes und 20% Natriumligninsulfonat in der Trockensubstanz enthalten, besprüht. Es werden 0,1, 0,05 und 0,02%ige Spritzbrühen (bezogen auf die Trockensubstanz) verwendet. Nach dem Abtrocknen des Spritzbelages werden die Blätter mit einer Zoosporenaufschwemmung von Plasmopara vitivola infiziert. Die Pflanzen kommen dann zuerst für 16 Stunden in eine wasserdampfgesättigte (feuchte) Kammer bei 20° C und anschließend 8 Tage in ein Gewächshaus mit Temperaturen zwischen 20 und 30° C. Nach dieser Zeit werden die Pflanzen zur Beschleunigung und Verstärkung des Sporangienträgerausbruches abermals während 16 Stunden in der feuchten Kammer aufgestellt. Dann wird eine Beurteilung des Krankheitsausbruches vorgenommen; hierbei bedeuten 0 = kein Pilzbefall, abgestuft bis 5 = Totalbefall (Kontrolle).

| Wirkstoff | Befall der Blätter nach Spritzung mit . . . %iger Wirkstoffbrühe 0,05 | 0,025 |
|---|---|---|
| 16 | 0 | 0 |
| 18 | 0 | 2 |
| 19 | 0 | 1 |
| Kontrolle (unbehandelt) | 5 | |

0 = kein Pilzbefall.
Abgestuft bis 5 = Blätter total befallen.

**Patentansprüche für die Vertragsstaaten BE, CH, DE, FR, GB, IT, LU, NL, SE**

1. α-Azolyl-α-phenyl-essigsäurederivate der Formel I

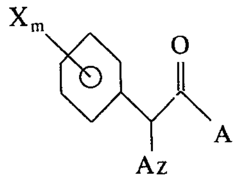

(I)

in der

X Wasserstoff, Fluor, Chlor oder Brom,
m 1, 2 oder 3
A die Gruppe OR,
worin R für eine Alkyl-, Cycloalkyl-, Alkenyl- oder Alkinylgruppe mit jeweils bis zu 12 Kohlenstoffatomen oder einen Aryl- oder Aralkylrest mit bis zu 12 Kohlenstoffatomen, welcher durch 1–3 Alkyl- oder Alkoxygruppen mit 1–4 Kohlenstoffatomen, Halogenatome, Trifluormethyl-, Cyano- und/oder Nitrogruppen substituiert sein kann, steht, oder
$NR^1R^2$,
worin $R^1$ und $R^2$ gleich oder verschieden sind und für Wasserstoffatome, Alkyl-, Cycloalkyl-, Alkenyl- oder Alkinylgruppen mit jeweils bis zu 12 Kohlenstoffatomen oder Aryl- oder Aralkylgruppen mit jeweils bis zu 12 Kohlenstoffatomen, die durch 1–3 Alkyl- oder Alkoxygruppen mit 1–4 Kohlenstoffatomen, Halogenatome, Trifluormethyl-, Cyano- und/oder Nitrogruppen substituiert sein können, stehen oder $R^1$ und $R^2$ zusammen mit N einen gesättigten Ring mit 4–8 Ringgliedern bilden, der zusätzlich ein Sauerstoff- oder Schwefelatom enthalten und durch 1–4 Halogenatome oder Alkylgruppen mit 1–4 Kohlenstoffatomen substituiert sein kann, und
Az einen Imidazol-1-yl- oder 1,2,4-Triazol-1-yl-rest bedeuten,
wobei jedoch A ein Rest $NR^1R^2$ sein muß, worin $R^1$ und $R^2$ keine Wasserstoffatome sind, wenn X Wasserstoff ist,

sowie deren Säureadditionssalze und Metallkomplexe.

2. α-(2,4-Dichlorphenyl)-α-(1,2,4-triazol-1-yl)-essigsäure-t-butylester.

3. Verbindung, ausgewählt aus der Gruppe bestehend aus

α-(2,4-Dichlorphenyl)-α-(1,2,4-triazol-1-yl)-essigsäure-N,N-diisopropyl-amid,
α-(2,4-Dichlorphenyl)-α-(1,2,4-triazol-1-yl)-essigsäure-N-cyclohexyl-N-methyl-amid,
α-(2,4-Dichlorphenyl)-α-(1,2,4-triazol-1-yl)-essigsäure-N-t-butyl-N-cyclohexyl-amid,
α-(2,4-Dichlorphenyl)-α-(1,2,4-triazol-1-yl)-essigsäure-N-1-butyl-N-isopropyl-amid,
α-(2,4-Dichlorphenyl)-α-(1,2,4-triazol-1-yl)-essigsäure-N-4-chlorphenyl-N-methyl-amid,
α-(2,4-Dichlorphenyl)-α-(1,2,4-triazol-1-yl)-essigsäure-N-isopropyl-N-phenyl-amid,
α-(2,4-Dichlorphenyl)-α-(1,2,4-triazol-1-yl)-essigsäure-N-2,6-dimethylpiperidid,
α-(2,4-Dichlorphenyl)-α-(1,2,4-triazol-1-yl)-essigsäure-N-t-butyl-N-methyl-amid,
α-Phenyl-α-(1,2,4-triazol-1-yl)-essigsäure-N,N-diisopropyl-amid,
α-(4-Chlorphenyl)-α-(1,2,4-triazol-1-yl)-essigsäure-N-methyl-N-but-1-in-3-yl-amid,
α-(4-Chlorphenyl)-α-(1,2,4-triazol-1-yl)-essigsäure-diisobutyl-amid.

4. Verfahren zur Herstellung der α-Azolyl-α-phenyl-essigsäurederivate der Formel I gemäß Anspruch 1, dadurch gekennzeichnet, daß man α-Halogensäurederivate der Formel III

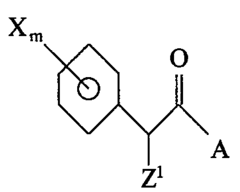

(III)

in der X, m und A die oben angegebene Bedeutung haben und $Z^1$ ein Chlor- oder Bromatom bedeutet, mit den Azolen H–Az, in denen Az die oben angegebene Bedeutung hat, ggf. in Gegenwart einer Base und/oder eines Lösungs- oder Verdünnungsmittels umsetzt, und die so erhaltenen Verbindungen ggf. in ihre Säureadditionssalze oder Metallkomplexe überführt.

5. Fungizid, enthaltend mindestens eine Verbindung gemäß Anspruch 1.

6. Fungizid, enthaltend mindestens eine Verbindung gemäß Anspruch 1 und einen festen oder flüssigen Trägerstoff.

7. Verfahren zur Bekämpfung von Fungi, dadurch gekennzeichnet, daß man mindestens ein α-Azolyl-α-phenylessigsäurederivat der Formel I gemäß Anspruch 1 auf diese einwirken läßt.

8. Verfahren zur Herstellung von Fungiziden, dadurch gekennzeichnet, daß man α-Azolyl-α-phenylessigsäurederivate der Formel I gemäß Anspruch 1 mit festen oder flüssigen Trägerstoffen mischt.

9. Verfahren zur vorbeugenden Bekämpfung von Fungi, dadurch gekennzeichnet, daß man mindestens ein Fungizid gemäß Anspruch 1 auf durch Fungi-Befall bedrohte Flächen, Pflanzen oder Saatgüter einwirken läßt.

**Patentansprüche für den Vertragsstaat AT**

1. Verfahren zur Herstellung von α-Azolyl-α-phenylessigsäurederivaten der Formel I

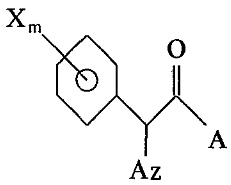

(I)

in der

X Wasserstoff, Fluor, Chlor oder Brom,
m 1, 2 oder 3
A die Gruppe OR,
worin R für eine Alkyl-, Cycloalkyl-, Alkenyl- oder Alkinylgruppe mit jeweils bis zu 12 Kohlenstoffatomen oder einen Aryl- oder Aralkylrest mit bis zu 12 Kohlenstoffatomen, welcher durch 1—3 Alkyl- oder Alkoxygruppen mit 1—4 Kohlenstoffatomen, Halogenatome, Trifluormethyl-, Cyano- und/oder Nitrogruppen substituiert sein kann, steht, oder
$NR^1R^2$,
worin $R^1$ und $R^2$ gleich oder verschieden sind und für Wasserstoffatome, Alkyl-, Cycloalkyl-, Alkenyl- oder Alkinylgruppen mit jeweils bis zu 12 Kohlenstoffatomen oder Aryl- oder Aralkylgruppen mit jeweils bis zu 12 Kohlenstoffatomen, die durch 1—3 Alkyl- oder Alkoxygruppen mit 1—4 Kohlenstoffatomen, Halogenatome, Trifluormethyl-, Cyano- und/oder Nitrogruppen substituiert sein können, stehen oder $R^1$ und $R^2$ zusammen mit N einen gesättigten Ring mit 4—8 Ringgliedern bilden, der zusätzlich ein Sauerstoff- oder Schwefelatom enthalten und durch 1—4 Halogenatome oder Alkylgruppen mit 1—4 Kohlenstoffatomen substituiert sein kann, und
Az einen Imidazol-1-yl- oder 1,2,4-Triazol-1-yl-rest bedeuten,
wobei jedoch A ein Rest $NR^1R^2$ sein muß, worin $R^1$ und $R^2$ keine Wasserstoffatome sind, wenn X Wasserstoff ist,

sowie deren Säureadditionssalze und Metallkomplexe, dadurch gekennzeichnet, daß man α-Halogensäurederivate der Formel III

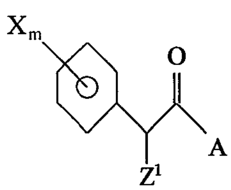

(III)

in der X, m und A die oben angegebene Bedeutung haben und $Z^1$ ein Chlor- oder Bromatom bedeutet, mit den Azolen H—Az, in denen Az die oben angegebene Bedeutung hat, ggf. in Gegenwart einer Base und/oder eines Lösungs- oder Verdünnungsmittels umsetzt, und die so erhaltenen Verbindungen ggf. in ihre Säureadditionssalze oder Metallkomplexe überführt.

2. Fungizid, enthaltend mindestens eine Verbindung der Formel I gemäß Anspruch 1.

3. Fungizid, enthaltend mindestens eine Verbindung der Formel I gemäß Anspruch 1 und einen festen oder flüssigen Trägerstoff.

4. Verfahren zur Bekämpfung von Fungi, dadurch gekennzeichnet, daß man mindestens ein α-Azolyl-α-phenylessigsäurederivat der Formel I gemäß Anspruch 1 auf diese einwirken läßt.

5. Verfahren zur Herstellung von Fungiziden, dadurch gekennzeichnet, daß man α-Azolyl-α-phenylessigsäurederivate der Formel I gemäß Anspruch 1 mit festen oder flüssigen Trägerstoffen mischt.

6. Verfahren zur vorbeugenden Bekämpfung von Fungi, dadurch gekennzeichnet, daß man mindestens ein Fungizid der Formel I gemäß Anspruch 1 auf durch Fungi-Befall bedrohte Flächen, Pflanzen oder Saatgüter einwirken läßt.

**Claims for the contracting states BE, CH, DE, FR, GB, IT, LU, NL, SE**

1. α-Azolyl-α-phenylacetic acid derivatives of the formula

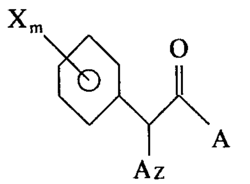

(I)

where X denotes hydrogen, fluorine, chlorine or bromine, m denotes 1, 2 or 3, A denotes OR, R denoting alkyl, cycloalkyl, alkenyl or alkynyl, each of up to 12 carbon atoms, or aryl or aralkyl of up to 12 carbon atoms which may or may not be substituted by 1 to 3 alkyl or alkoxy groups of 1 to 4 carbon atoms, halogen trifluoromethyl, cyano and/or by nitro, or A denotes $NR^1R^2$, $R^1$ and $R^2$ being identical or different and each denoting hydrogen, alkyl, cycloalkyl, alkenyl or alkynyl, each of up tc 12 carbon atoms, or aryl or aralkyl of a maximum of 12 carbon atoms which may or may not be substituted by 1 to 3 alkyl or alkoxy groups of 1 to 4 carbon atoms, halogen, trifluoromethyl, cyano and/or by nitro, or $R^1$ and $R^2$ together with N forming a saturated ring with 4 to 8 ring members which may additionally contain an oxygen or sulfur atom and may or may not be substituted by 1 to 4 halogen atoms or alkyl groups of 1 to 4 carbon atoms, and Az denotes imidazol-1-yl or 1,2,4-triazol-1-yl, A being, when X is hydrogen, $NR^1R^2$, $R^1$ and $R^2$ not being hydrogen, and their acid addition salts and metal complexes.

2. α-(2,4-Dichlorophenyl)-α-(1,2,4-triazol-1-yl)-acetic acid-t-butyl ester.

3. A compound selected from the group consisting of

α-(2,4-dichlorophenyl)-α-(1,2,4-triazol-1-yl)-acetic acid-N,N-diisopropyl amide,
α-(2,4-dichlorophenyl)-α-(1,2,4-triazol-1-yl)-acetic acid-N-cyclohexyl-N-methyl amide,
α-(2,4-dichlorophenyl)-α-(1,2,4-triazol-1-yl)-acetic acid-N-t-butyl-N-cyclohexyl amide,
α-(2,4-dichlorophenyl)-α-(1,2,4-triazol-1-yl)-acetic acid-N-t-butyl-N-isopropyl amide,
α-(2,4-dichlorophenyl)-α-(1,2,4-triazol-1-yl)-acetic acid-N-4-chlorophenyl-N-methyl amide,
α-(2,4-dichlorophenyl)-α-(1,2,4-triazol-1-yl)-acetic acid-N-isopropyl-N-phenyl amide,
α-(2,4-dichlorophenyl)-α-(1,2,4-triazol-1-yl)-acetic acid-N-2,6-dimethylpiperidide,
α-(2,4-dichlorophenyl)-α-(1,2,4-triazol-1-yl)-acetic acid-N-t-butyl-N-methyl amide,
α-phenyl-α-(1,2,4-triazol-1-yl)-acetic acid-N,N-diisopropyl amide,
α-(4-chlorophenyl)-α-(1,2,4-triazol-1-yl)-acetic acid-N-methyl-N-but-1-yn-3-yl amide,
and α-(4-chlorophenyl)-α-(1,2,4-triazol-1-yl)-acetic acid-diisobutyl amide.

4. A process for the manufacture of α-azolyl-α-phenylacetic acid derivatives of the formula I as claimed in Claim 1, characterized in that an α-halocarboxylic acid derivative of the formula III

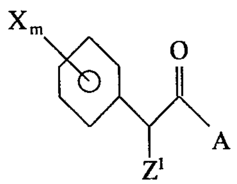

(III)

where X, m and A have the above meanings and $Z^1$ denotes a chlorine or bromine atom, is reacted with an azole H−Az, where Az has the above meanings, in the presence or absence of a base and/or solvent or diluent, and the compound thus obtained is, if desired, converted into an acid addition salt or metal complex.

5. A fungicide containing at least one compound as claimed in claim 1.

6. A fungicide containing at least one compound as claimed in claim 1 and a solid or liquid carrier.

7. A process for combating fungi, characterized in that at least one α-azolyl-α-phenylacetic acid derivative of the formula I as claimed in claim 1 ist allowed to act thereon.

8. A process for producing a fungicide, characterized in that an α-azolyl-α-phenylacetic acid derivative of the formula I as claimed in claim 1 is mixed with a solid or liquid carrier.

9. A process for the prophylatic control of fungi, characterized in that at least one fungicide as claimed in claim 1 is allowed to act on areas, plants or seed threatened by fungus attack.

**Claims for the contracting state AT**

1. A process for the manufacture of α-azolyl-α-phenylacetic acid derivatives of the formula

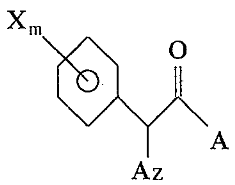

(I)

where X denotes hydrogen, fluorine, chlorine or bromine, m denotes 1, 2 or 3, A denotes OR, R denoting alkyl, cycloalkyl, alkenyl or alkynyl, each of up to 12 carbon atoms, or aryl or aralkyl of up to 12 carbon atoms which may or may not be substituted by 1 to 3 alkyl or alkoxy groups of 1 to 4 carbon atoms, halogen, trifluoromethyl, cyano and/or by nitro, or A denotes $NR^1R^2$, $R^1$ and $R^2$ being identical or different and each denoting hydrogen, alkyl, cycloalkyl, alkenyl or alkynyl, each of up to 12 carbon atoms, or aryl or aralkyl of up to 12 carbon atoms which may or may not be substituted by 1 to 3 alkyl or alkoxy groups of 1 to 4 carbon atoms, halogen, trifluoromethyl, cyano and/or by nitro, or $R^1$ and $R^2$ together with N forming a saturated ring with 4 to 8 ring members which may additionally contain an oxygen or sulfur atom and may or may not be substituted by 1 to 4 halogen atoms or alkyl groups of 1 to 4 carbon atoms, and Az denotes imidazol-1-yl or 1,2,4-triazol-1-yl, A being, when X ist hydrogen, $NR^1R^2$, $R^1$ and $R^2$ not being hydrogen, and their acid addition salts and metal complexes, characterized in that an α-halocarboxylic acid derivative of the formula III

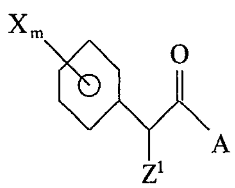

(III)

where X, m and have the above meantings and $Z^1$ denotes a chlorine or bromine atom, is reacted with an azole H−Az has the above meanings, in the presence or absence of a base and/or solvent or diluent, and the compound thus obtained is, if desired, converted into an acid addition salt or metal complex.

2. A fungicide containing at least one compound of the formula I as claimed in claim 1.

3. A fungicide containing at least one compound of the formula I as claimed in claim 1 and a solid or liquid carrier.

4. A process for combating fungi, characterized in that at least one α-azolyl-α-phenylacetic acid derivative of the formula I as claimed in claim 1 is allowed to act thereon.

5. A process for producing a fungicide, characterized in that an α-azolyl-α-phenylacetic acid derivative of the formula I as claimed in claim 1 ist mixed with a solid or liquid carrier.

6. A process for the prophylatic control of fungi, characterized in that at least one fungicide of the formula I as claimed in claim 1 ist allowed to act on areas, plants or seed threatened by fungus attack.

**Revendications pour les Etats contractonts BE, CH, DE, FR, GB, IT, LU, NL, SE**

1. Dérivés d'acide α-azolyl-α-phénylacétique de formule I

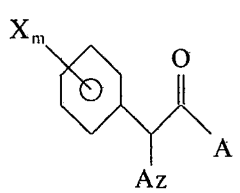

(I)

dans laquelle

X représente hydrogène, fluor, chlore ou brome,
m 1, 2 ou 3
A les groupes OR,
où R représente un groupe alkyle, cycloalkyle, alcényle ou alcinyle, ayant chacun jusqu'à 12 atomes de carbone, ou un reste aryle ou aralkyle ayant jusqu'à 12 atomes de carbone, qui peut être substitué par 1−3 groupes alkyle ou alkoxy à 1−4 atomes de carbone, un atome d'halogene,

des groupes trifluorométhyle, cyano et/ou nitro, ou
$NR^1R^2$,
où $R^1$ et $R^2$ sont identiques ou différents et représentent un atome d'hydrogène, les groupes alkyle, cycloalkyle, alcényle ou alcinyle ayant chacun jusqu'à 12 atomes de carbone ou des groupes aryle ou aralkyle ayant chacun jusqu'à 12 atomes de carbone, qui peuvent être substitués par 1—3 groupes alkyle ou alcoxy à 1—4 atomes de carbone, atome d'halogène, des groupes trifluorométhyle, cyano, et/ou nitro, ou bien $R^1$ et $R^2$ ensemble avec N forment un noyau saturé à 4—8 chaînons, qui contient — en outre, un atome d'oxygène ou de soufre et peut être substitué par 1—4 atomes d'halogène ou des groupes alkyle à 1—4 atomes de carbone, et
Az représente un reste imidazol-1-yle ou 1,2,4-triazol-1-yle, A devant être un reste $NR^1R^2$, où $R^1$ et $R^2$ ne sont pas des atomes d'hydrogène lorsque X est l-hydrogène

ainsi que leurs sels d'addition d'acides et leurs complexes métalliques.

2. t-butylester d'acide α-(2,4-dichlorophényl)-α-(1,2,4-triazol-1-yl)-acétique.

3. Composé choisi dans le groupe constitué de

N,N-diisopropyl-amide d'acide α-(2,4-dichlorophényl)-α-(1,2,4-triazol-1-yl)-acétique,
N-cyclohexyl-N-méthyl-amide d'acide α-(2,4-dichlorophényl)-α-(1,2,4-triazol-1-yl)-acétique,
N-t-butyl-N-cyclohexyl-amide d'acide α-(2,4-dichlorophényl)-α-(1,2,4-triazol-1-yl)-acétique,
N-1-butyl-N-isopropyl-amide d'acide α-(2,4-dichlorophényl)-α-(1,2,4-triazol-1-yl)-acétique,
N-4-chlorophényl-N-méthyl-amide d'acide α-(2,4-dichlorophényl)-α-(1,2,4-triazol-1-yl)-acétique,
N-isopropyl-N-phényl-amide d'acide α-(2,4-dichlorophényl)-α-(1,2,4-triazol-1-yl)-acétique,
N-2,6-diméthylpipéridide d'acide α-(2,4-dichlorophényl)-α-(1,2,4-triazol-1-yl)-acétique,
N-t-butyl-N-méthyl-amide d'acide α-(2,4-dichlorophényl)-α-(1,2,4-triazol-1-yl)-acétique,
N,N-diisopropyl-amide d'acide α-(phényl-α-(1,2,4-triazol-1-yl)-acétique,
N-méthyl-N-but-1-in-3-yl-amide d'acide α-(4-chlorophényl)-α-(1,2,4-triazol-1-yl)-acétique,
Diisobutyl-amide d'acide α-(4-chlorophényl)-α-(1,2,4-triazol-1-yl)-acétique.

4. Procédé de préparation de dérivés d'acides α-azolyl-α-phénylacétique de formule I, selon la revendication 1, caractérisé par le fait que l'on fait réagir des dérivés d'acides α-halogénés de formule III

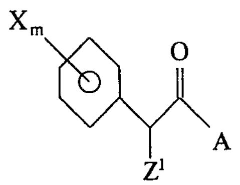

(III)

dans laquelle X, m et A ont la signification indiquée plus haut et $Z^1$ représente un atome de chlore ou de brome, avec les azoles H—Az, dans lequel Az a la signification indiquée plus haut, éventuellement en présence d'une base et/ou d'un solvant ou diluant, et les composés ainsi obtenus sont éventuellement transformés en leurs sels d'addition d'acides ou complexes métalliques.

5. Fongicide, contenant au moins un composé selon la revendication 1.

6. Fongicide, contenant au moins un composé selon la revendication l et un support solide ou liquide.

7. Procédé de lutte contre les champignons, caractérisé par le fait que l'on fait agir sur ceux-ci au moins un dérivé d'acide α-azolyl-α-phénylacétique de formule I selon la revendication 1.

8. Procédé de préparation de fongicides, caractérisé par le fait qu'on mélange des dérivés d'acide α-azolyl-α-phénylacétique de formule I selon la revendication 1 avec des supports solides ou liquides.

9. Procédé pour la lutte préventive contre les champignons, caractérisé par le fait qu'on fait agir au moins un fongicide selon la revendication l sur des surfaces, plantes ou semences, menacées d'attaque par des champignons.

**Revendications pour l'Etat contractant AT**

1. Procédé de préparation de dérivés d'acide α-azolyl-α-phénylacétique de formule I

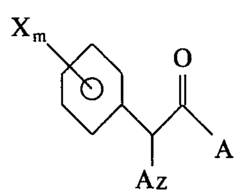

(I)

dans laquelle

X représente hydrogéne, fluor, chlore ou brome,
m 1, 2 ou 3
A les groupes OR,
où R représente un groupe alkyle, cycloalkyle, alcényle ou alcinyle, ayant chacun jusqu'à 12 atomes de carbone, ou un reste aryle ou aralkyle ayant jusqu'à 12 atomes de carbone, qui peut être substitué par 1—3 groupes alkyle ou alkoxy à 1—4 atomes de carbone, un atome d'halogène, des groupes trifluorométhyle, cyano et/ou nitro, ou
$NR^1R^2$,
où $R^1$ et $R^2$ sont identiques ou différents et représentent un atome d'hydrogène, les groupes alkyle, cycloalkyle, alcényle ou alcinyle ayant chacun jusqu'à 12 atomes de carbone ou des groupes aryle ou aralkyle ayant chacun jusqu'à 12 atomes de carbone, qui peuvent être substitués par 1—3 groupes alkyle ou alcoxy à 1—4 atomes de carbone, atome d'halogène, des groupes trifluorométhyle, cyano, et/ou nitro, ou bien $R^1$ et $R^2$ ensemble avec N forment un noyau saturé à 4—8 chaînons, qui contient, — en outre, un atome d'oxygène ou de soufre et peut être substitué par 1—4 atomes d'halogène ou des groupes alkyle à 1—4 atomes de carbone, et
Az représente un reste imidazol-1-yle ou 1,2,4-triazol-1-yle, A devant être un reste $NR^1R^2$, où $R^1$ et $R^2$ ne sont pas des atomes d'hydrogène lorsque X est l'hydrogène

ainsi que leurs sels d'addition d'acides et leurs complexes métalliques, caractérisé par le fait que l'on fait réagir des dérivés d'acides $\alpha$-halogénés de formule III

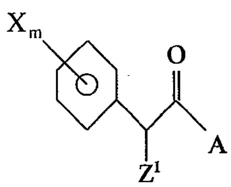

(III)

dans laquelle X, m et A ont la signification indiquée plus haut et $Z^1$ représente un atome de chlore ou de brome, avec les azoles H—Az, dans lequel Az a la signification indiquée plus haut, éventuellement en présence d'une base et/ou d'un solvant ou diluant, et les composés ainsi obtenus sont éventuellement transformés en leurs sels d'addition d'acides ou complexes métalliques.

2. Fongicide, contenant au moins un composé de formule I selon la revendication 1.

3. Fongicide, contenant au moins un composé de formule I selon la revendication 1 et un support solide ou liquide.

4. Procédé de lutte contre les champignons, caractérisé par le fait que l'on fait réagir sur ceux-ci au moins un dérivé d'acide $\alpha$-azolyl-$\alpha$-phénylacétique de formule I selon la revendication 1.

5. Procédé de préparation de fongicides, caractérisé par le fait qu'on mélange des dérivés d'acide $\alpha$-azolyl-$\alpha$-phénylacétique de formule I selon la revendication 1 avec des supports solides ou liquides.

6. Procédé pour la lutte préventive contre les champignons, caractérisé par le fait qu'on fait agir au moins un fongicide de formule I selon la revendication 1 sur des surfaces, plantes ou semences, menacées d'attaque par des champignons.